# EUROPEAN PATENT APPLICATION

(11) **EP 4 287 210 A1**
(43) Date of publication of application: **06.12.2023**
(21) Application number: 22177106.6
(22) Date of filing: 02.06.2022
(51) Int. Cl.: G16H 50/20, G16H 20/17, G16H 40/63, A61B 5/00

(54) **DEVICE, COMPUTERIZED METHOD, MEDICAL SYSTEM FOR
DETERMINING A PREDICTED VALUE OF GLYCEMIA**

(71) Applicant: Diabeloop, 38028 Grenoble Cedex 1 (FR)
(72) Inventor: ADENIS, Alice, 38340 Voreppe (FR); LOUIS, Maxime, 38000 Grenoble (FR); ROMERO-UGALDE, Hector, 38420 Le Versoud (FR); DAUDET, Laurent, 38410 Saint Martin d Uriage (FR)
(74) Representative: Fidal Innovation

(57) **Abstract**

A device for determining a predicted value of glycemia, the device including a data processing unit (3) adapted to process time-based data. The data processing unit (3) is configured to implement a temporal predictive model trained to learn a glycemia cyclic temporal behaviour, where said glycemia cyclic temporal behaviour is defined by variations of glycemia values in a cyclic way over a given cyclic period of time.The temporal predictive model is configured to:receive, as inputs:
▪ said time-based data; and
▪ encoded temporal data, said encoded temporal data being encoded as to represent the given cyclic period of time.

The temporal predictive model is also configured to deliver, as output, a predicted value of glycemia.

## Description

### FIELD OF THE INVENTION

The present invention relates to a device and method for determining a glycemia cyclic temporal behaviour over a given cyclic period of time, and a medical system including such device.

### BACKGROUND OF THE INVENTION

In the field of health care, and more precisely in the field of treating diabetes, it is known to evaluate the concentration of blood glucose, also called glycemia, to inject a quantity of insulin as a function of the measured concentration.

Recently, so-called "closed-loop" systems were developed, where a processor is programmed to evaluate a volume rate of insulin to be injected, based on patient-related data and/or time-based data, such as past and/or present measures of glycemia, and to control the injection of insulin based on this evaluation. In addition, the processor can be programmed to evaluate a volume of insulin to be injected in some special circumstances, in particular meals, and/or physical activity. The quantity can be injected to the patient, subject to the patient's approval. Such systems are also called "semi closed-loop" systems because of the necessary declaration by the patient of some of these special circumstances.

The time-based data is often used to predict the future concentration of glycemia. This prediction is then used to calculate the quantity of insulin having to be injected in order to maintain the concentration of blood glucose in acceptable intervals.

An incorrect prediction of the future blood glucose can lead to an irrelevant calculated quantity of insulin to be injected, leading to a concentration of blood glucose in unacceptable intervals, where the patient may be in hypoglycemia and/or hyperglycemia.

In the scientific paper [Van Doom et al. 2021] W. P. van Doom, Y. D. Foreman, N. C. Schaper, H. H. Savelberg, A. Koster, C. J. van der Kallen, A. Wesselius, M. T. Schram, R. M. Henry, P. C. Dagnelie, et al., "Machine learning-based glucose prediction with use of continuous glucose and physical activity monitoring data: The maastricht study," PloS one, vol. 16, no. 6, p. e0253125, 2021, it is described a method for predicting a glucose blood concentration in a closed-loop system. The method predicts a future glucose value with a machine learning model trained to predict future glucose levels based on prior CGM (Continuous Glucose Monitoring). The method of this paper predicts glucose values at intervals of 15 and 60 minutes by a machine learning model that has been trained with time-based data being a sliding time window of glucose values preceding the predicted values at a fixed interval.

One main drawback of this method is that the method does not enable taking into account a general behaviour of the glycemia, influenced, for example, by the patient's habit. This leads to a prediction of a future glycemia lacking accuracy.

Another scientific paper [Mhaskar et al. 2017] H. N. Mhaskar, S. V. Pereverzyev, and M. D. van der Walt, "A deep learning approach to diabetic blood glucose prediction," Frontiers in Applied Mathematics and Statistics, vol. 3, p. 14, 2017, describes a method for 30-minutes prediction of blood glucose levels measured by continuous glucose monitoring devices, using clinical data set. The clinical data set lists blood glucose levels of patients taken at 5-min intervals with the continuous glucose monitoring device. A deep-learning model is trained to predict future blood glucose level, based on time-based data being the time series of blood glucose level at each time interval.

One main drawback of this method is that the method does not enable taking into account a general behaviour of the glycemia, influenced, for example, by the patient's habit. This leads to a prediction of a future glycemia lacking accuracy.

The invention thus aims to answer at least partially the above presented technical problems.

### BRIEF SUMMARY OF THE INVENTION

Thus, the invention relates to a device for determining a predicted value of glycemia, the device including a data processing unit adapted to process time-based data, wherein:
➢ the data processing unit is configured to implement a temporal predictive model trained to learn a glycemia cyclic temporal behaviour, where said glycemia cyclic temporal behaviour is defined by variations of glycemia values in a cyclic way over a given cyclic period of time,
➢ the temporal predictive model being configured to:
   ∘ receive, as inputs:
      ▪ said time-based data; and
      ▪ encoded temporal data, said encoded temporal data being encoded as to represent the given cyclic period of time;
   ∘ deliver, as output, a predicted value of glycemia.

*Thanks to these provisions, the temporal predictive model is able to take into account a general behaviour of the glycemia, influenced, for example, by the patient's habit. This leads to an accurate prediction* of a *future glycemia.*

According to different aspects, it is possible to provide the characteristics below taken alone or in combination.

According to an embodiment, at least a part of the encoded temporal data is encoded according to a linear distribution over the given cyclic period of time according to a predetermined constant time step.

*According to this provision, the injected encoded data may be a time point in the time interval, the time point corresponding to the present time, i.e. the time the encoded temporal data is injected in the predictive temporal model. With this configuration, the temporal predictive model has access to all the information needed to learn the glycemia tendency over the given period of time and hence predict an accurate future value of glycemia.*

According to an embodiment, at least a part of the encoded temporal data is encoded according to an angular distribution over the given cyclic period of time according to a predetermined constant angle interval.

*In the embodiment where the encoded temporal data is encoded according to a linear distribution, the predictive temporal model could have difficulty understanding the relationship between two consecutive given cyclic periods of time due to the discontinuous encoding. The angular encoding of the encoded temporal data may use the sine and cosine functions, since the sine and cosine functions constitute a continuous and periodic isometry of the cyclic periods of time. That way, the encoding fully preserves distances between any two times in the cyclic periods. This embodiment then constitutes a more principled encoding of the time information.*

According to an embodiment, the time-based data includes at least one past glycemia value.

*The injection of past glycemia value and the encoded temporal data are sufficient for the temporal predictive model to output accurate predicted glycemia values.*

According to an embodiment, the temporal predictive model is further configured to receive as an input:
➢ a variable IOB(t) representative of the time variation of the patient's quantity of insulin on board; and
➢ a variable COB(t) representative of the time variation of the patient's quantity of carbohydrate on board.

*Since these parameters may have an influence on glycemia, the injection of the IOB(t) and the COB(t) in the temporal predictive model may help enhance even more the value of the predicted glycemia.*

According to an embodiment, the given cyclic period of time is a day, a week and/or a year.

*Several cyclic periods of time may then be considered, leading to a robust and flexible device for glycemia prediction.*

According to an embodiment, the encoded temporal data is tagged according to:
➢ a night-time and/or a day-time when the given cyclic period of time is a day;
➢ a particular day of the week when the given cyclic period of time is a week;
➢ a working day and/or a week-end period or a holiday when the given cyclic period of time is a week; and/or
➢ a day of the year when the given cyclic period of time is a year.

Such configuration may allow the temporal predictive model to take into account behavioural properties during the given cyclic period. For instance:
➢ during the night the glycemia tends to be more stable;
➢ during workdays meal patterns and stress level are different from weekends;
➢ meal contents may vary with the season.

The present application also relate to a computerized method for determining a predicted value of glycemia, said method including a data processing unit processing at least time-based data, wherein:
said data processing unit implements a temporal predictive model trained to learn a glycemia cyclic temporal behaviour, where said glycemia cyclic temporal behaviour is defined by variations of glycemia values in a cyclic way over a given cyclic period of time;
wherein the temporal predictive model:
   ➢ receives, as inputs:
      ∘ said at least time-based data; and
      ∘ encoded temporal data, said encoded temporal data being encoded as to represent the given cyclic period of time;
   ➢ delivers, as an output, a predicted value of glycemia.

In an embodiment, the method includes a prior learning phase including:
➢ acquiring a plurality of at least time-based data for a plurality of patients;
➢ injecting said plurality of time-based data and the encoded temporal data in the temporal predictive model;
➢ training said temporal predictive model until it converges;
➢ storing said temporal predictive model.

*The learning phase of the temporal predictive model with a plurality of time-based data, from a plurality of persons may increase the robustness of the temporal predictive model since it may be able to learn a plurality of different glycemia variations and behaviours.*

According to an embodiment, the method includes a prior learning phase including:
➢ acquiring a plurality of time-based data for one patient;
➢ injecting said plurality of time-based data and the encoded temporal data in the temporal predictive model;
➢ training said temporal predictive model until it converges;
➢ storing said temporal predictive model.

*This configuration allows the temporal predictive model to know in a really accurate way the glycemia temporal behavior of one person since its training is personalised, with the data* of *said person.*
the present application also relates to a medical system for regulating a glycemia of a person including:
➢ a device according to the present application, said device storing the trained temporal predictive model;
➢ a medical device wearable by a patient, including:
   ∘ a dispenser of insulin, controllable according to the predicted value of glycemia.

In an embodiment, the medical device further includes:
➢ a data acquisition system configured to acquire at least time-based data;
➢ a communication module configured to repeatedly transmit the acquired time-based data to the data processing unit of the device.

*Such configuration allows the temporal predictive model, once trained, to receive the at least time-based data of the patient wearing the medical device, such that the temporal predictive model can be used to help patients treating their diabete.*

In an embodiment, the device is embedded in the medical device.

The present application also relates to a computer program including instructions which, when the program is executed by a computer, cause the computer to carry out a determination of a predicted value of glycemia of a device including a data processing unit adapted to process time-based data, wherein:
➢ the data processing unit is configured to implement a temporal predictive model trained to learn a glycemia cyclic temporal behaviour, where said glycemia cyclic temporal behaviour is defined by variations of glycemia values in a cyclic way over a given cyclic period of time; wherein the temporal predictive model is configured to:
➢ receive, as inputs:
   ∘ said at least time-based data; and
   ∘ encoded temporal data, said encoded temporal data being encoded as to represent the given cyclic period of time;
➢ deliver, as an output, a predicted value of glycemia.

### BRIEF DESCRIPTION OF THE DRAWINGS

Embodiments of the invention will be described below with reference to the drawings, described briefly below:
[Fig. 1] schematically shows a medical system according to one embodiment of the invention.
[Fig. 2] represents a diagram illustrating a data processing unit according to one embodiment.
[Fig. 3] represents some steps of a method for training a temporal predictive model according to one embodiment.
[Fig. 4] represents some steps of a method for training a temporal predictive model according to another embodiment.

In the drawings, identical references designate identical or similar objects.

### DETAILED DESCRIPTION OF THE INVENTION

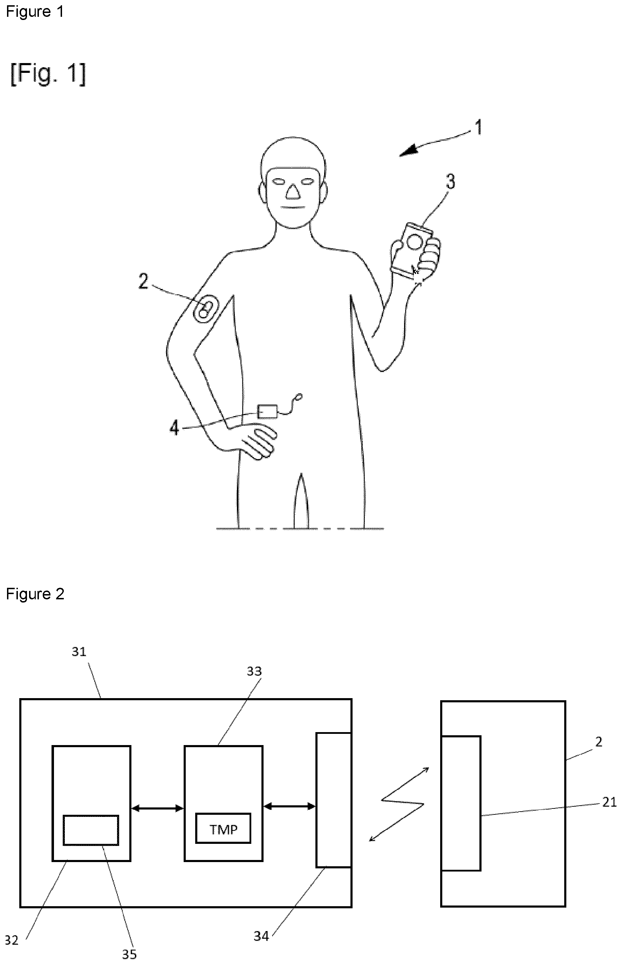

Figure 1 shows a medical system 1. The medical system 1 is advantageously used in the field of diabetes.

The medical system 1 typically includes a data acquisition system 2, a data processing unit 3 and a drug dispensing system 4, notably an insulin dispensing system 4.

The medical system 1 is a computerized medical system 1. In particular, the data processing unit 3 is a computerized data processing unit. Typically, the data acquisition system 2 includes a computerized data acquisition system. The insulin dispensing system 4 may be a computerized insulin dispensing system 4, including a processor which performs a process based on input parameters.

Any computerized system may include a processing subsystem, a storage subsystem, a user interface, a communication subsystem and a power subsystem. A computerized system may also include other components such as power battery, connectors and so on (not explicitly shown).

A storage subsystem can store data. In some embodiments, a storage subsystem can also store one or more computer programs to be executed by a processing system or a processing subsystem.

The medical system 1 may also include a user interface (not shown). The patient may be able to enter some data through the user interface, as will be described in more details below. The user interface may also be configured to deliver outputs to the patient, such as notifications.

Examples of user interface may include a screen, speakers, etc.

The data acquisition system 2 of the medical system 1 is adapted to measure a parameter of the user such as time-based data. The data acquisition system 2 may be a Continuous Glucose Monitoring (CGM) sensor. In particular, the data acquisition system 2 might be wearable by a patient. In addition, the data acquisition system 2 is adapted to repeatedly measure time-based data of the patient. In an example, the measure is made at each time step, the time step being between 1 minute and 15 minutes, preferably every 5 minutes.

Time is associated with the measured data by means of a clock of the data acquisition system 2. Such a data acquisition system may be called a continuous data acquisition system. The data acquisition system 2 may be adapted to perform in vivo measurements

The time-based data may be a past and/or present concentration of glucose, also called glycemia.

The data acquisition system 2 includes a data communication module 21 adapted to communicate data to the data processing unit 3. The data communication module 21 might be adapted to communicate wirelessly, for example using a short-range radio communication corresponding to the Bluetooth^{®} standard or BLE standard.

The data processing unit 3 of the medical system 1 may include a computerized system 31 for communicating treatment information to a user. Said computerized system 31, as shown on figure 2, includes a processor 32 and a memory 33. The data processing unit 3 includes a data communication module 34 adapted to communicate data, i.e. to receive and transmit data. The data communication module 34 might be adapted to communicate wirelessly, for example using a short-range radio communication corresponding to the Bluetooth^{®} standard. For example, the data communication module 34 of the terminal 3 and the data communication module 21 of the data acquisition system 2 are compatible with one another, to form a communication channel, by which the data processing unit 3 and the data acquisition system 2 communicate with one another. In particular, time-based data is communicated from the sensing unit 2 to the data processing unit 3. The time-based data may be stored in the memory 22.

As seen on figure 1, the medical system 1 further includes an insulin dispensing system 4.

The insulin dispensing system 4 includes a container 41 configured to contain insulin. Typically, the container 41 is able to contain a plurality of insulin doses, so as to be used a plurality of times. The insulin dispensing device 4 further includes a dispensing unit 42 adapted to dispense drug to the user. It for example includes a needle to be inserted into the body of the user, and a plunger 43 which can be pushed to expel insulin from the container 41.

The insulin dispensing system 4 may further include a setting unit 44. The setting unit 44 can be set by the user in order to control the amount of insulin which may be injected at a time using the insulin dispensing device 4. For example, the setting unit 44 may be set by the user to dispense a certain amount of insulin. The dispensing system is then actuated to dispense the set amount of insulin and is prevented from operating more insulin than the set amount. For example, a settable mechanical stop for the plunger 8 may be used.

Other embodiments are possible. For example, the setting unit 44 is the user interface, through which the patient is able to set an amount of insulin. According to this configuration, the insulin dispensing system 4 includes a communication interface (not shown) able to communicate with the user interface.

In an embodiment, the data acquisition system 2, the data processing unit 3 and the insulin dispensing system 4 may communicate to each other through wireless communication, such as the Bluetooth standard. In another embodiment, they communicate with each other through wired connections.

In an embodiment, the data acquisition system 2, the data processing unit 3, the insulin dispensing system 4 and the user interface may communicate to each other through wireless communication, such as the Bluetooth standard. In another embodiment, they communicate with each other through wired connections.

In an embodiment, the data processing unit 3 could be integrated within the same unit as the data acquisition system 2 in a medical device, wearable by a patient.

In a variant embodiment, the data processing unit 3 could be integrated within the same unit as the insulin dispensing device 4, in a medical device wearable by a patient.

In yet a variant embodiment, the data acquisition system 2 could be integrated within the same unit as the insulin dispensing device 4. Thus, according to one embodiment, the data acquisition system 2, the processing system 3 and the insulin dispensing device 4 may be integrated within the same medical device wearable by a patient.

In a further embodiment, the data acquisition system 2, the data processing unit 3, the insulin dispensing system 4 and the user interface may be integrated within the same medical device wearable by a patient.

Figure 2 more specifically shows an example of the data processing unit 3.

The data processing unit 3 is configured to, at least, process data acquired by the data acquisition system 2.

The data processing unit 3 includes a computerized system 31 including a processor 32 and a memory 33 storing data. In particular, the memory stores time-based data, acquired at least in part by the data acquisition system 2. The time-based data acquired by the data acquisition system 2 are transmitted to the memory 33 of the computerized system 31, via the data communication interface 34.

The memory 33 may also store patient-related data and other possible data, as meal-related data. Such data may be transmitted by the patient, via the user interface (not shown).

The data-processing system 3 is a computerized system which operates data-processing according to an artificial-intelligence-based scheme. In an embodiment, the processor 32 implements a temporal predictive model, designated as TPM on figure 2. The temporal predictive model is trained to determine a glycemia cyclic temporal behavior so as to be able to predict a future value of glycemia. The trained temporal predictive model may be stored in the memory 33.

The result of the data-processing is an output. In the medical system 1, the output can include a predicted value of the glycemia, i.e. a value of the glycemia at a future time. More particularly, the output can include a predicted value of the glycemia of the patient using the medical system.

The outputted predicted value of glycemia can be processed by a determination module 35 of the processor 32, configured to determine an insulin dose to be injected to the patient, based on the predicted value of glycemia at the future time. Such determined insulin dose may then be used as a control parameter to the insulin dispensing system 4.

The determined insulin dose may be subjected to the patient's setting, as described above.

The implemented temporal predictive model may be a machine-learning model or a deep-learning model. The temporal predictive model may use a recurrent neural network.

As stated above, the temporal predictive model is trained to determine a glycemia cyclic temporal behavior over a predetermined cyclic period of time and to deliver, as an output, a predicted value of glycemia.

Generally, the temporal predictive model may receive, as input, at least one time-based data. Such a configuration allows the predictive model to accurately predict a glycemia taking into account a general behaviour of the glycemia, influenced, for example, by the patient's habit.

The time-based data includes, at least, past and/or present values of the glycemia of the patient. The past and/or present values of the glycemia are past and/or present regarding the time the prediction of the future glycemia is outputted.

The time-based data may also include insulin-on-board IOB(t) which is a variable representative of the time variation of the patient's quantity of insulin on board, and carbs-on-board COB(t) which is a variable COB(t) representative of the time variation of the patient's quantity of carbohydrate on board. Such a configuration allows the temporal predictive model to more accurately predict the glycemia as the IOB(t) and the COB(t) may both have an impact on the glycemia.

Other predefined time-based data may be taken into account and may allow the temporal predictive model to be more accurate, as long as the data contains information relative to the current physiology of the patient. For example, the sensitivity to insulin defines how the patient reacts to insulin intakes, and would prove valuable for the predictions accuracy.

These parameters are called time-based data since their amount might change with time during the given cyclic time period. In other words, these parameters can be expressed as a function of time.

The temporal predictive model may also receive patient-related data as input. The patient-related data may include a delivered insulin quantity parameter, a consumed carbohydrate quantity parameter and patient-specific parameters such an Insulin Sensitivity Factor, or a Carbohydrate-to-Insulin Ratio parameter. The consumed carbohydrate quantity parameter represents a carbohydrate quantity ingested by the patient.

The temporal predictive model may also receive, as input, meal-related data, which may correspond to a quantity of carbohydrate ingested by the patient.

Since the patient-related data and meal-related data are variables that may have an influence on the glycemia, it may be useful to inject them in the temporal predictive model to obtain a more accurate prediction of a future glycemia.

The time-based data may be enough for the temporal predictive model to predict a future glycemia value. However, the prediction may be enhanced when the temporal model also receives as input the patient-related data and/or the meal-related data.

Finally, the temporal predictive model receives, as input, encoded temporal data.

The encoded temporal data represents the given cyclic time period. Hence the temporal data may be encoded according to several forms to represent the given cyclic time period.

One advantage is that time is taken into account by the predictive temporal model, such that it may help the temporal predictive model to learn glycemia temporal behaviour during a cyclic period of time. The temporal predictive model may then be able to learn the patient's habits such that a glycemia behaviour will be linked to a same time, or at least a same time period, included in the given cyclic period of time. A predicted value of the glycemia at a future time may then be predicted with more accuracy.

The given cyclic time period may correspond either to a day, a week and/or a year.

The temporal predictive model may be trained to determine the glycemia cyclic behaviour at each hour of the day, at each day of a week and/or at each day of a year.

These are not limiting examples.

One advantage of this configuration is that a glycemia general tendency over a relatively long period of time can be known with more accuracy than in the state of art.

In an embodiment, the temporal data is encoded using a linear transformation over the given cyclic time period with a predetermined constant time step. In this embodiment, the temporal predictive model receives as inputs a sequence of time-based data over the given period of time, sampled with a constant time step, as well as one or several (e.g. hour, day of the week, day of the year) encoded temporal data corresponding to one time point of the given period of time.

In a particular embodiment, the time point received by the temporal predictive model corresponds to the present time.

Generally, such encoded temporal data may take the following form:
**{x ∈** 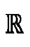 **| a <= x < b} = [a, b[,** where **a, x** and **b** are real numbers, **[a, b[** is the interval, a is the beginning of the given cyclic period of time, **b** is the end of the given cyclic period of time, **x** is a time point of the given cyclic period of time, between **a** and **b.**

Hence, when the present time is **x,** the temporal predictive model will receive **x** as the encoded temporal data. When the present time is **x+1,** the temporal predictive model will receive **x + 1** as the encoded temporal data.

As stated above, at least one time-based data is sampled and inputted in the temporal predictive model at a time step between 1 minute and 20 minutes, and preferably every 5 minutes. Hence, if the predetermined constant time step between two time points of the time interval of the encoded data is greater than 20 minutes, and preferably greater than 5 minutes, the encoded temporal data and the time-based data may not be constantly injected in the temporal predictive model at the same time. In other words, the encoded temporal data may not be injected as an input in the temporal predictive model each time the temporal predictive model receives the time-based data.

More particularly, in the embodiment where the given cyclic period of time is a day and the temporal predictive model is trained to determine the glycemia cyclic behaviour at each hour of the day, the encoded temporal data injected in the temporal predictive model may take the following form:

**{x ∈** 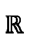 **| 0 <= x < 24} = [0, 24[,** where 0 is the beginning of the given cyclic period of time, meaning the first hour of a day (for example 1am), **24 (or 23 or 25 for time change)** is the end of the given cyclic period of time, meaning the last hour of a day (for example midnight), and **x** is an hour of the day between **0** and **24.** Then, the constant time step separating each point of the interval is one hour.

In this example, the time-based data will, for example, be injected as an input in the temporal predictive model every five minutes, while the encoded temporal data corresponding to the present time are injected as an input in the temporal predictive model every one hour.

In the embodiment where the given cyclic period of time is a week and the temporal predictive model is trained to determine the glycemia cyclic behaviour at each day of the week, the encoded temporal data injected in the temporal predictive model may take the following form:
**{x ∈** 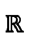 **| 0 <= x < 7} = [0, 7[,** where **0** is the beginning of the given cyclic period of time, meaning the first day of a week (it could be any day of the week, for example Monday), **7** is the end of the given cyclic period of time, meaning the last day of the week (for example Sunday when the first day is Monday), and **x** is a day of the week between **0** and **7.** Then, the constant time step separating each point of the interval is one day.

In this example, the time-based data will, for example, be injected as an input in the temporal predictive model every five minutes, while the encoded temporal data corresponding to the present day of the week are injected as an input in the temporal predictive model every day.

In the particular embodiment where the given cyclic period of time is a year and the temporal predictive model is trained to determine the glycemia cyclic behaviour at each day of the year, the encoded temporal data injected in the temporal predictive model may take the following form:
**{x ∈** 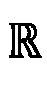 **| 0** <= **x** < **365}** = **[0, 365],** (or 366 for the bissextile years) where **0** is the beginning of the given cyclic period of time, meaning the first day of the year (it could be, for example, January 1^{st}), **365** is the end of the given cyclic period of time, meaning the last day of the year (for example December 31^{st}), and **x** is a day of the year between **0** and **365.** Then, the constant time step separating each point of the interval is one day.

In this example, the time-based data will, for example, be injected as an input in the temporal predictive model every five minutes, while the encoded temporal data corresponding to the present day of the year are injected as an input in the temporal predictive model every day.

These embodiments are not limiting examples, and other given cyclic period of time and hence other time steps between two consecutive time points of the interval could be considered.

As an example, more precise encoding of the temporal data could be done, for example by diminishing the time step between two points in each interval. In particular, instead of an hour of a day, a minute of a day could be considered as a time step, in which each time points x of the considered interval would be spaced not according to an hour, as in the above described embodiment, but be spaced according to a minute to have a better and more accurate representation of the given cyclic period of time.

Also, the temporal predictive model could be trained with encoded temporal data representing a night time versus a daytime, such that the temporal predictive model would be able to differentiate a glycemia cyclic temporal behaviour during day and night.

The temporal predictive model could also be trained with encoded temporal data representing a working day period versus a week-end or holiday period.

In yet another embodiment, the encoded temporal data may be injected in the temporal predictive model each time the at least one time-based data is injected. This may be useful when the time-based data are irregularly sampled.

Although the embodiment of the encoding of the temporal data described above is adequate when taking into account the time in the prediction of a cyclic temporal glycemia behaviour, it may lead to some inaccuracy for some time points of the intervals.

Indeed, to determine a glycemia cyclic temporal behaviour over a given cyclic period of time, the temporal predictive model would be trained on several consecutive given periods of time, for example on consecutive several days. Hence, several temporal data encoded according to the linear distribution described above would be injected in the temporal predictive model in conjunction with at least the time-based data, and possibly the patient-related data and the meal-related data. However, the linear distribution of the temporal data described above is discrete, meaning that there is no continuity between the end b of a first temporal data representative of a given cyclic period of time, and the beginning a of another temporal data representative of a consecutive given cyclic period of time.

As an illustration, for two consecutive days, the last time point x of a first day would be 24 and the first time point **x** of a second day would be zero. For the predictive temporal model, there is then a great difference between 24 and 0, on the contrary there is no such great difference between the last hour of a day and the first hour of a consecutive day (literally only one minute).

It results from the above that, due to the discrete encoding of the temporal data in this particular embodiment, the predictive temporal model could struggle to digest that there is a continuity between two consecutive given cyclic periods of time. It is especially true for the time points near the beginning and end of each consecutive several cyclic periods of time.

Another embodiment of an encoding of temporal data may therefore be implemented.

According to this second embodiment, the temporal data is encoded according to an angular distribution over the given cyclic period of time according to a predetermined constant angle interval.

Generally, such encoded temporal data takes the following form:
**(cos((x) / n * 2pi), (sin((x) / n * 2pi),** where **x** is a time point of the given cyclic period of time and n is the total amount of time points of the given cyclic period of time. Each time point x is separated from the other by a constant angular value.

Hence, in this embodiment the temporal data is encoded according to a cosine function and a sine function. Both functions are injected in the temporal predictive model as inputs.

Given this encoding, the continuity between two consecutive given cyclic time periods is ensured. More particularly, since the sine and cosine functions are continuous, the temporal data encoded according to the sine and cosine functions are also continuous. Then, the same time step separates each time point of the given cyclic period of time, as well as the last time point of the given cyclic period of time and the first time point of a consecutive cyclic period of time. In other words, the temporal data is encoded according to a continuous curve.

Furthermore, the temporal data is encoded with both the cosine and the sine functions since with this combined use they define a unique angular value, while with the use of only a cosine or a sine function, each angle could take two different values.

In a more particular way, when the given cyclic period of time is a day, and the temporal predictive model is trained to determine the glycemia cyclic behaviour at each hour of the day, the temporal data would be encoded according to the following:
**(cos((x) / N * 2pi), (sin((x) / N * 2pi),** where **x** is the hour of the day of one time point in the given cyclic period between 0 and N is the number of hours in the given day (24 or 23 or 25 for time change days). Hence, according to this example, each hour of a day is represented by a unique angular value.

An advantage of using this set is that it allows to use sampling times that are not constant. For example, an instant t of the day may be timestamped with a precision up to the nanosecond, using the above encoding with x the hour of the day where x is a real number (for instance, x=4.35 corresponds to 4h21). If measurements are randomly sampled throughout the day, this embodiment still contains the information of the instant of the day, therefore the distance between two encoded points are consistent with the time spent in reality.

When the given cyclic period of time is a week, and the temporal predictive model is trained to determine the glycemia cyclic behaviour at each day of the week, the temporal data would be encoded according to the following:
**(cos((x) / 7 * 2pi), (sin((x) / 7 * 2pi),** where **x** is the day of the week of one time point in the given cyclic period between 0 and 7. Hence, according to this example, each day of the week is represented by a unique angular value.

When the given cyclic period of time is a year, and the temporal predictive model is trained to determine the glycemia cyclic behaviour at each day of the year, the temporal data would be encoded according to the following:
**(cos((x) / D * 2pi), (sin((x) / D * 2pi),** where **x** is the day of the year of one time point in the given cyclic period between 0 and D, where **D** is the total number of days in the year (365 or 366). Hence, according to this example, each day of the year is represented by a unique angular value.

These embodiments are not limiting examples, and other given cyclic periods of time and hence other time encodings could be considered, such as a day of a month.

Also, more precise encoding of the temporal data could be done. For example, in the embodiment where the given cyclic time period is a day, the total amount of hours (24) could be replaced with the total amount of minutes in one day. Hence, each time point x would correspond to a minute of a day, and each angular value would represent one minute of a day.

Other encoding of temporal data representing a given cyclic period of time can be considered which may enter within the scope of the present application.

For example, as stated above, the temporal predictive model could be trained with encoded temporal data representing a night time versus a daytime, such that the temporal predictive model would be able to differentiate a glycemia cyclic temporal behaviour during day and night.

The temporal predictive model could also be trained with encoded temporal data representing a working day period versus a week-end period.

Whatever the encoding of the temporal data is, the injection of such encoded temporal data as an input to the temporal predictive model allows the temporal predictive model to determine a glycemia cyclic behaviour over this cyclic period of time.

This can be done since both temporal data and the other inputs, i.e. at least the patient-related data and possibly the time-based data and the meal-related data, are injected in the temporal predictive model. This enables the temporal predictive model to consider and take into account the patient habits and the circadian cycle of a patient to output a predicted value of a future glycemia.

More precisely, regarding the patient's habit, a person generally has the same general behaviour over a given cyclic period of time.

For example concerning a day, a person will notably have the same habits regarding a time of meal, a time of sleep, of waking up, etc. By adding the time as an input, the temporal predictive model is able to link the set of data to the temporal data such that the temporal predictive model knows, eventually, how the glycemia is going to evolve during a day relative to the time of the day.

The same can be applied regarding a week, a month and/or a year.

Furthermore, the training of the temporal predictive model regarding the night time or the daytime can help the temporal predictive model to understand or at least consider the circadian cycle of the patient.

In an embodiment, all the encoded temporal data, and more generally all the inputs of the temporal predictive model, are tagged during the training of the temporal predictive model.

Due to the injection of the encoded temporal data in the temporal predictive model, the temporal predictive model may eventually be able to discern particular times of interest over the given cyclic period of time. Typically, such particular times of interest may be defined by the glycemia behaviour which evolves according to a similar pattern. Such patterns and hence such particular times of interest may be linked to the patient's habits.

As a non-limitative illustration, each day a patient may generally take their first meal around 8am, the second meal around 12am and the last meal around 6pm. The patient may go to bed generally around 10pm and wake up around 7am. According to what is known about glycemia evolution, the glycemia will highly increase during a meal time and may present a low variability during the sleeping time. Hence, by injecting temporal data in the temporal predictive model, the temporal predictive model will be able to determine particular times of interest representative of meal times, in this example the particular times of interest would be around 8am, 12am and 6pm when the glycemia will highly increase.

The temporal predictive model may also be able to determine a particular time of interest representative of the sleeping time during which the glycemia slightly varies, in this example between 10pm and 7am.

This arrangement has two main technical advantages. First, the accuracy of the predicted value of glycemia is enhanced since the patient's habits are considered.

Second, an action can be taken regarding the outputted predicted glycemia value and the sub period associated with it. In particular, an unexpected variation in glycemia can be considered as abnormal considering the sub period in which the variation takes place.

Moreover, according to previous studies, some parameters having an impact on glycemia are known to change during the day following a circadian cycle. Injecting time as an input to the temporal predictive model may then enable to have a better understanding of this phenomenon.

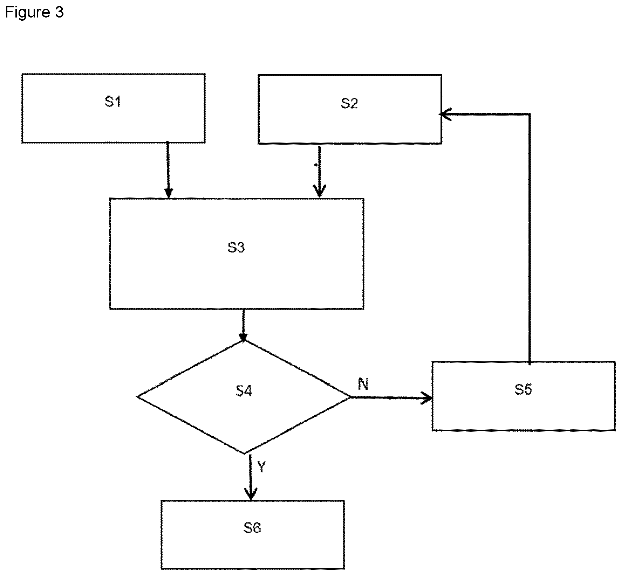

Figure 3 illustrates the training of a temporal predictive model according to a first embodiment.

At step S1, the temporal predictive model receives as inputs at least the time-based data, being a present and/or past glycemia value(s). The inputs may also include patient related data and/or meal-related data.

According to this embodiment, the temporal predictive model receives a plurality of time-based data, and optionally patient-related data and meal-related data, of a plurality of patients.

In the embodiment of figure 3, the time-based data are acquired on a plurality of patients, by a data acquisition system 2. The time-based data, the patient-related data and the meal-related data may be stored in a database and injected in the temporal predictive model during its training.

In another embodiment, the time-based data, the patient-related data and the meal-related data of the plurality of patients come from a database in which a plurality of illustrating data are stored. These data may not have been acquired on patients but may be equivalent to acquired data, to effectively train the temporal predictive model.

At step S2, the temporal predictive model receives as input the encoded temporal data representative of the given cyclic period of time. The encoded temporal data may be encoded according to the embodiments described above.

The inputs of the temporal predictive model may be tagged.

At step S3, the temporal predictive model is trained. At step S4, it is determined if the temporal predictive model has converged.

If the temporal predictive model has not converged at step S4, steps S1 and S2 are implemented again for the plurality of patients (step S5).

If the temporal predictive model has converged, it is stored at step S6.

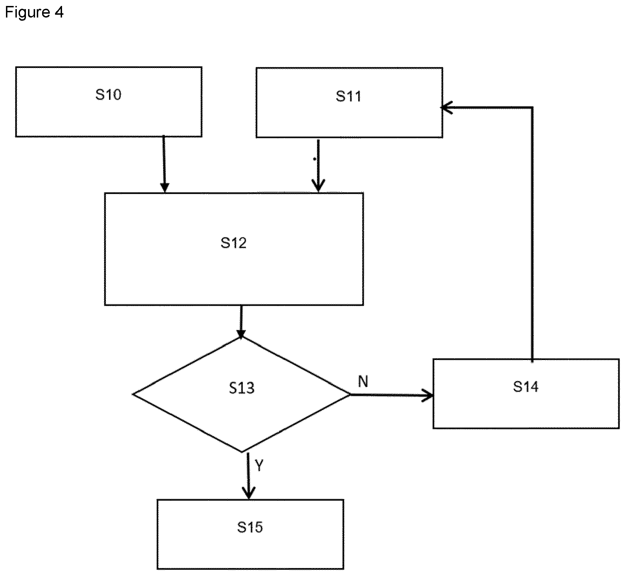

Figure 4 illustrates another embodiment of the training of the temporal model.

At step S10, the temporal predictive model receives as inputs at least the time-based data. As explained above, the data may also include patient related data and/or meal-related data.

According to this embodiment, the temporal predictive model receives a plurality of time-based data of one patient. The temporal predictive model may also receive a plurality of patient-related data and/or meal-related data of the same patient. Advantageously, the patient from which the data is injected in the temporal predictive model is the patient who wears the medical system 1 storing the temporal predictive model once it is trained.

At step S11, the temporal predictive model receives as input the encoded temporal data representative of the given cyclic period of time. The encoded temporal data may be encoded according to the embodiments described above.

The inputs of the temporal predictive model may be tagged.

At step S12, the temporal predictive model is trained. At step S13, it is determined if the temporal predictive model has converged.

If the temporal predictive model has not converged at step S13, steps S10 and S11 are implemented again for the same patient (step S14).

At step S15, the temporal predictive model is stored if it has converged.

For both of the embodiments described in relation with figures 3 and 4, the temporal predictive model may be stored in the memory 33 of the data processing unit 3 of the medical system 1.

In an alternative embodiment, the trained temporal predictive model may be stored on a remote server communicating with the data processing unit 3 of the medical system 1.

Once trained, the temporal predictive model receives time-based data from the patient wearing the medical device 1. The time-based data are acquired by the acquisition system 2. The temporal predictive model may also receive patient-related data, inputted by the patient via the user interface. The temporal predictive model also receives the encoded temporal data.

While exemplary embodiments of the invention has been described, it will be understood by those skilled in the art that various changes, omissions and/or additions may be made, and equivalents may be substituted for elements thereof without departing from the spirit and scope of the invention. In addition, many modifications may be made to adapt a particular situation or material to the teachings of the invention without departing from the scope thereof. Therefore, it is intended that the invention is not limited to the particular embodiments disclosed for carrying out this invention, but that the invention will include all embodiments falling within the scope of the appended claims. Moreover, unless specifically stated any use of the terms first, second, etc. do not denote any order or importance, but rather the terms first, second, etc. are used to distinguish one element from another.

## Claims

1. A device for determining a predicted value of glycemia, the device including a data processing unit (3) adapted to process time-based data, wherein:
➢ the data processing unit (3) is configured to implement a temporal predictive model trained to learn a glycemia cyclic temporal behaviour, where said glycemia cyclic temporal behaviour is defined by variations of glycemia values in a cyclic way over a given cyclic period of time,
➢ the temporal predictive model being configured to:
∘ receive, as inputs:
▪ said time-based data; and
▪ encoded temporal data, said encoded temporal data being encoded as to represent the given cyclic period of time;
∘ deliver, as output, a predicted value of glycemia.

2. Device according to claim 1, wherein at least a part of the encoded temporal data is encoded according to a linear distribution over the given cyclic period of time according to a predetermined constant time step.

3. Device according to claim 1 or 2, wherein at least a part of the encoded temporal data is encoded according to an angular distribution over the given cyclic period of time according to a predetermined constant angle interval.

4. Device according to claims 1 to 3, wherein the time-based data includes at least:
➢ one past glycemia value.

5. Device according to claims 1 to 4, wherein the temporal predictive model is further configured to receive as input:
➢ a variable IOB(t) representative of the time variation of the patient's quantity of insulin on board; and
➢ a variable COB(t) representative of the time variation of the patient's quantity of carbohydrate on board.

6. Device according to any of claims 1 to 5, wherein the given cyclic period of time is:
➢ a day;
➢ a week; and/or
➢ a year.

7. Device according to claim 6, wherein the encoded temporal data is tagged according to:
➢ a night-time and/or a day-time when the given cyclic period of time is a day;
➢ a particular day of the week when the given cyclic period of time is a week;
➢ a working day and/or a week-end day when the given cyclic period of time is a week; and/or
➢ a day of the year when the given cyclic period of time is a year.

8. A computerized method for determining a predicted value of glycemia, said method including a data processing unit (3) processing at least time-based data, **wherein:**
➢ said data processing unit (3) implements a temporal predictive model trained to learn a glycemia cyclic temporal behaviour, where said glycemia cyclic temporal behaviour is defined by variations of glycemia values in a cyclic way over a given cyclic period of time;
➢ wherein the temporal predictive model:
∘ receives, as inputs:
▪ said at least time-based data; and
▪ encoded temporal data, said encoded temporal data being encoded as to represent the given cyclic period of time;
➢ delivers, as output, a predicted value of glycemia.

9. Computerized method according to claim 8, including a prior learning step including:
➢ acquiring a plurality of at least time-based data for a plurality of patients;
➢ injecting said plurality of time-based data and the encoded temporal data in the temporal predictive model;
➢ training said temporal predictive model until it converges;
➢ storing said temporal predictive model.

10. Computerized method according to claim 8, including a prior learning step including:
➢ acquiring a plurality of time-based data for one patient;
➢ injecting said plurality of time-based data and the encoded temporal data in the temporal predictive model;
➢ training said temporal predictive model until it converges;
➢ storing said temporal predictive model.

11. Medical system (1) for regulating a glycemia of a person including:
➢ a device according to any of claims 1 to 7, said device including a memory storing the trained temporal predictive model according to claim 9 or 10;
➢ a medical device wearable by a patient, including:
∘ a dispenser of insulin (4), controllable according to the predicted value of glycemia.

12. Computerized medical system (1) according to claim 11, wherein the medical device further includes:
➢ a data acquisition system (2) configured to acquire at least time-based data;
➢ a communication module configured to repeatedly transmit the acquired time-based data to the data processing unit (3) of the device.

13. Computerized medical system (1) according to any of claims 11 or 12, wherein the device according to any of claims 1 to 7 is embedded in the medical device.

14. A computer program including instructions which, when the program is executed by a computer, cause the computer to carry out a determination of a predicted value of glycemia of a device including a data processing unit (3) adapted to process time-based data, **wherein:**
➢ the data processing unit (3) is configured to implement a temporal predictive model trained to learn a glycemia cyclic temporal behaviour, where said glycemia cyclic temporal behaviour is defined by variations of glycemia values in a cyclic way over a given cyclic period of time;
➢ wherein the temporal predictive model is configured to:
∘ receive, as inputs:
▪ said at least time-based data; and
▪ encoded temporal data, said encoded temporal data being encoded as to represent the given cyclic period of time;
∘ deliver, as output, a predicted value of glycemia.
